# EUROPEAN PATENT APPLICATION

(11) **EP 3 508 125 A1**
(43) Date of publication of application: **10.07.2019**
(21) Application number: 18215699.2
(22) Date of filing: 21.12.2018
(51) Int. Cl.: A61B 6/00, A61B 6/03

(54) **METHOD AND APPARATUS FOR PERFORMING COMPUTED TOMOGRAPHY (CT) IMAGING BY INJECTING CONTRAST MEDIUM**

(30) Priority: 27.12.2017 KR 20170181522
(71) Applicant: Samsung Electronics Co., Ltd., Suwon-si, Gyeonggi-do, 16677 (KR)
(72) Inventor: Lee, Changlae, 16677 Suwon-si, Gyeonggi-do (KR); Jang, Wooyoung, 16677 Suwon-si, Gyeonggi-do (KR); Jung, Jinwook, 16677 Suwon-si, Gyeonggi-do (KR); Choi, Yuna, 16677 Suwon-si, Gyeonggi-do (KR)
(74) Representative: Gulde & Partner

(57) **Abstract**

The method of performing CT imaging by injecting the contrast medium into the object includes: setting a region of interest (ROI) in a scout image of the object; acquiring X-ray projection images by detecting X-rays that have passed through the ROI; measuring a difference between pixel values of at least one X-ray projection image acquired before an injection of a contrast medium into the ROI and pixel values of at least one X-ray projection image acquired after the injection of the contrast medium into the ROI; and determining whether to initiate a diagnostic CT scan by comparing the measured difference with a preset threshold.

## Description

### BACKGROUND

### 1. Field

The disclosure relates to methods and apparatuses for performing CT imaging by injecting a contrast medium into an object, and more particularly, to methods and apparatuses for initiating a CT scan by tracking an optimal time point of contrast enhancement based on acquired projection images of an object.

### 2. Description of Related Art

Medical imaging apparatuses are used to acquire images showing an internal structure of an object. The medical imaging apparatuses are non-invasive examination apparatuses that capture and process images of details of structures, tissue, fluid flow, etc., inside a body and provide the images to a user. A user, e.g., a medical practitioner, may use medical images output from the medical imaging apparatuses to diagnose the patient's condition and diseases.

A CT apparatus is an example of an apparatus for imaging an object by emitting X-rays toward a patient. The CT apparatus is capable of providing a cross-sectional image of an object and may represent an internal structure (e.g., organs such as a kidney, a lung, etc.) of the object without superimposition of adjacent structures, as compared to a typical X-ray apparatus. Due to these advantages, the CT apparatus is widely used for diagnostic purposes.

CT angiography is used to obtain a CT image of a blood vessel such as a carotid artery, a pulmonary artery, etc., via a CT apparatus by injecting a contrast medium into the blood vessel. In CT imaging using injection of a contrast medium, a bolus tracking technique is used to more clearly visualize a region of interest (ROI), i.e., a blood vessel, by tracking a time point at which a contrast effect due to the contrast medium is maximized. A related art bolus tracking method involves acquiring a pre-scan image by reconstructing an X-ray projection image generated by emitting X-rays toward an object into which a contrast medium is injected and determining a time point for initiation of a diagnostic CT scan by measuring a Hounsfield Unit (HU) value in an ROI that is set in the acquired pre-scan image. In detail, the bolus tracking method is achieved by monitoring whether an HU value in the ROI is greater than a preset threshold and determining the start of a diagnostic CT scan at a time point when the HU value is greater than the preset threshold.

However, according to the bolus tracking method, a user suffers the inconvenience of having an ROI individually set in a pre-scan image, and when the ROI is set in a wrong region other than a region to be observed in the pre-scan image, a CT scan has to be performed again. Furthermore, it takes a long time to acquire a pre-scan image by reconstructing an X-ray projection image, and the user may miss a time point at which an attenuation coefficient for a contrast medium reaches its maximum value during reconstruction of the pre-scan image. In addition, since an X-ray source emits X-rays while rotating on a rotating frame through 360 degrees to acquire a pre-scan image, the amount of radiation to which a patient is exposed may be excessive.

### SUMMARY

Provided are CT methods and apparatuses for determining an optimal time point for initiating a diagnostic CT scan by using pixel values in at least one X-ray projection image respectively obtained before and after injection of a contrast medium into an object.

Additional aspects will be set forth in part in the description which follows and, in part, will be apparent from the description, or may be learned by practice of the presented embodiments.

In accordance with an aspect of the disclosure, a method of performing CT imaging by injecting a contrast medium into an object includes: setting an ROI in a scout image of the object; acquiring at least one X-ray projection image by detecting X-rays that have passed through the ROI; measuring a difference between pixel values of at least one X-ray projection image acquired before injection of a contrast medium into the ROI and at least one X-ray projection image acquired after the injection of the contrast medium into the ROI; and determining whether to initiate a diagnostic CT scan by comparing the measured difference with a preset threshold.

The method may further include acquiring the scout image by emitting X-rays toward a predetermined region of the object including the ROI.

The acquiring of the at least one X-ray projection image may include: emitting, via an X-ray source, the X-rays toward the ROI at at least one angular position; and acquiring the at least one X-ray projection image by detecting, by using an X-ray detector, the X-rays that are emitted at the at least one angular position and pass through the ROI.

The measuring of the difference between the pixel values may include: setting, as a reference value, a sum of pixel values of the at least one X-ray projection image acquired before the injection of the contrast medium into the ROI; calculating a difference between the reference value and a sum of pixel values of the at least one X-ray projection image acquired at each of a plurality of time points following the injection of the contrast medium into the ROI; and determining, among the plurality of time points, a time point when the calculated difference exceeds the preset threshold. The determining of whether to initiate the diagnostic CT scan may include determining initiation of the diagnostic CT scan for examining the object at the determined time point.

The setting of the reference value may include setting, as the reference value, a sum of pixel values of an X-ray projection image acquired at a first angular position from among the at least one X-ray projection image acquired before the injection of the contrast medium into the ROI. The calculating of the difference may include adding up, for each of the plurality of time points, pixel values of the X-ray projection image acquired at the first angular position from among the at least one X-ray projection image acquired at the plurality of time points following the injection of the contrast medium into the ROI and calculating a difference between a result of the adding up and the reference value.

The acquiring of the at least one X-ray projection image may include detecting the X-rays that have passed through the ROI by using a photon counting detector configured to detect X-rays having a plurality of energy bands.

The acquiring of the at least one X-ray projection image may include detecting, by using the photon counting detector, X-rays having an energy corresponding to a first energy band among the plurality of energy bands and acquiring the at least one X-ray projection image by using the detected X-rays.

The acquiring of the at least one X-ray projection image may include detecting X-rays having an energy band corresponding to a k-edge that is in an energy band in which an attenuation coefficient of the contrast medium increases rapidly among the plurality of energy bands and acquiring the at least one X-ray projection image by using the detected X-rays.

The acquiring of the at least one X-ray projection image may include: emitting X-rays from a plurality of X-ray sources having different tube voltages; and acquiring a plurality of X-ray projection images respectively corresponding to the plurality of energy bands by detecting, via a plurality of X-ray detectors, the X-rays that have passed through the ROI. The measuring of the difference between the pixel values may include measuring a difference between a reference value, which is a sum of pixel values of the at least one X-ray projection image acquired before the injection of the contrast medium into the ROI, and a sum of pixel values of an X-ray projection image acquired, among the plurality of X-ray projection images, by detecting X-rays having an energy corresponding to an energy band in which an attenuation coefficient of the contrast medium undergoes a largest variation.

The method may further include separating, from the acquired at least one X-ray projection image, a contrast projection image generated by detecting X-rays that have passed through the contrast medium. The measuring of the difference between the pixel values may include: setting, as a reference value, a sum of pixel values of the at least one X-ray projection image acquired before the injection of the contrast medium into the ROI; and calculating a difference between a sum of pixel values of the contrast projection image and the reference value.

The method may further include calculating an average of values of a preset number of adjacent pixels among pixels of the acquired at least one X-ray projection image and replacing the values of the preset number of adjacent pixels with the calculated average.

In accordance with an aspect of the disclosure, a CT apparatus includes: an X-ray source configured to emit X-rays toward an ROI of an object at at least one angular position arranged around the object; an X-ray detector configured to detect the X-rays that have passed through the ROI at the at least one angular position; a data acquisition unit configured to acquire at least one X-ray projection image by using the X-rays detected by the X-ray detector; and a processor configured to set the ROI in a scout image of the object, measure a difference between pixel values of at least one X-ray projection image acquired before injection of a contrast medium into the ROI and at least one X-ray projection image acquired after the injection of the contrast medium into the ROI, and determine whether to initiate a diagnostic CT scan by comparing the measured difference with a preset threshold.

The X-ray source may be further configured to emit X-rays toward a predetermined region of the object including the ROI, the X-ray detector may be further configured to detect the X-rays that have passed through the predetermined region of the object, and the processor may be further configured to generate the scout image by using the detected X-rays.

The processor may be further configured to set, as a reference value, a sum of pixel values of the at least one X-ray projection image acquired before the injection of the contrast medium into the ROI, calculate a difference between the reference value and a sum of pixel values of the at least one X-ray projection image acquired at each of a plurality of time points following the injection of the contrast medium into the ROI, and determine initiation of the diagnostic CT scan for examining the object at a time point when the calculated difference exceeds the preset threshold among the plurality of time points.

The processor may be further configured to set, as the reference value, a sum of pixel values of an X-ray projection image acquired at a first angular position from among the at least one X-ray projection image acquired before the injection of the contrast medium into the ROI, add up, for each of the plurality of time points, pixel values of the X-ray projection image acquired at the first angular position from among the at least one X-ray projection image acquired at the plurality of time points following the injection of the contrast medium into the ROI, and calculate a difference between a result of the adding up and the reference value.

The X-ray detector may be a photon counting detector (PCD) configured to detect X-rays having a plurality of energy bands.

The X-ray detector may be further configured to detect X-rays having an energy corresponding to a first energy band among the plurality of energy bands, and the data acquisition unit may be further configured to acquire the at least one X-ray projection image by using the X-rays detected by the X-ray detector.

The X-ray detector may be further configured to detect X-rays having an energy band corresponding to a k-edge that is in an energy band in which an attenuation coefficient of the contrast medium increases rapidly among the plurality of energy bands, and the data acquisition unit may be further configured to acquire the at least one X-ray projection image by using the X-rays detected by the X-ray detector.

The X-ray source may include a plurality of X-ray sources configured to emit X-rays at different tube voltages, and the X-ray detector may include a plurality of X-ray detectors configured to detect the X-rays that are respectively emitted by the plurality of X-ray sources and pass through the ROI. The data acquisition unit may be further configured to acquire a plurality of X-ray projection images respectively corresponding to the plurality of energy bands by using the X-rays detected by the plurality of X-ray detectors, and the processor may be further configured to measure a difference between a pixel value for an X-ray projection image, which is acquired, among the plurality of X-ray projection images, by detecting X-rays having an energy corresponding to an energy band in which an attenuation coefficient of the contrast medium undergoes a largest variation, and a pixel value for the at least one X-ray projection image acquired before the injection of the contrast medium into the ROI.

The processor may be further configured to separate, from the acquired at least one X-ray projection image, a contrast projection image including only X-rays detected when passing through only the contrast medium, set, as a reference value, a sum of pixel values of the at least one X-ray projection image acquired before the injection of the contrast medium into the ROI, and calculate a difference between a sum of pixel values of the contrast projection image and the reference value.

In accordance with an aspect of the disclosure, a computer program product includes a computer-readable recording medium including instructions for performing: setting an ROI in a scout image of the object; acquiring at least one X-ray projection image by detecting X-rays that have passed through the ROI; measuring a difference between pixel values of at least one X-ray projection image acquired before injection of a contrast medium into the ROI and at least one X-ray projection image acquired after the injection of the contrast medium into the ROI; and determining whether to initiate a diagnostic CT scan by comparing the measured difference with a preset threshold.

### BRIEF DESCRIPTION OF THE DRAWINGS

The above and other aspects, features, and advantages of certain embodiments of the present disclosure will be more apparent from the following description, taken in conjunction with the accompanying drawings, in which:
FIG. 1 is a conceptual diagram of a method, performed by a CT apparatus, of determining a time point to start a diagnostic CT scan, according to an embodiment;
FIG. 2 is a block diagram illustrating components of a CT apparatus according to an embodiment;
FIG. 3 is a flowchart of a method, performed by a CT apparatus, of determining a time point to start a diagnostic CT scan is performed, according to an embodiment;
FIG. 4 is a flowchart of a method, performed by a CT apparatus, of determining a time point to start a diagnostic CT scan, according to an embodiment;
FIG. 5 is a diagram of a method, performed by a CT apparatus, of determining a time point to start a diagnostic CT scan, according to an embodiment;
FIG. 6 is a graph of attenuation coefficients of a bone of an object, water, and a contrast medium, with respect to energy;
FIG. 7A is a block diagram showing components of a CT apparatus according to an embodiment;
FIG. 7B is a diagram of a method, performed by the CT apparatus, of performing a CT scan according to an embodiment;
FIG. 7C is a graph of the number of photons contained in X-rays, which are emitted by the CT apparatus, pass through an object, and are detected, with respect to an energy band;
FIGS. 8A, 8B, and 8C are conceptual diagrams of methods, performed by CT apparatus, of obtaining a plurality of X-ray projection images, according to embodiments;
FIG. 9 is a flowchart of a method, performed by a CT apparatus, of separating a contrast projection image from an X-ray projection image of an object, according to an embodiment;
FIG. 10 illustrates a method, performed by a CT apparatus, of binning pixels in an X-ray projection image such that noise in the X-ray projection image is reduced, according to an embodiment;
FIG. 11 is a diagram of a method of setting an ROI in a scout image of an object by using a CT apparatus, according to an embodiment;
FIG. 12 is a flowchart of a method, performed by a CT apparatus, of performing a diagnostic CT scan on an object, according to an embodiment; and
FIG. 13 illustrates a structure of a CT system according to an embodiment.

### DETAILED DESCRIPTION

Throughout the specification, like reference numerals or characters refer to like elements. In the present specification, all elements of embodiments, general matters in the technical field of the disclosure and redundant matters between embodiments are not described. Terms 'part' and 'portion' used herein may be implemented using software or hardware, and, according to embodiments, a plurality of 'parts' or 'portions' may be implemented using a single unit or element, or a single 'part' or 'portion' may be implemented using a plurality of units or elements. Expressions such as "at least one of," when preceding a list of elements, modify the entire list of elements and do not modify the individual elements of the list. For example, the expressions "at least one of a, b, and c" and "at least one of a, b, or c" should be understood as including only a, only b, only c, both a and b, both a and c, both b and c, or all of a, b, and c.

In the present specification, an image may include a medical image obtained by a medical imaging apparatus, such as a CT apparatus, a magnetic resonance imaging (MRI) apparatus, an ultrasound imaging apparatus, or an X-ray apparatus.

Throughout the specification, the term 'object' is a thing to be imaged, and may include a human, an animal, or a part of a human or animal. For example, the object may include a part of a body (i.e., an organ), a phantom, or the like.

In the present specification, a 'CT system' or 'CT apparatus' refers to a system or apparatus configured to emit X-rays while rotating around at least one axis relative to an object and image the object by detecting the X-rays.

In the specification, a 'CT image' refers to an image constructed from raw data obtained by imaging an object by detecting X-rays that are emitted as the CT system or apparatus rotates about at least one axis with respect to the object.

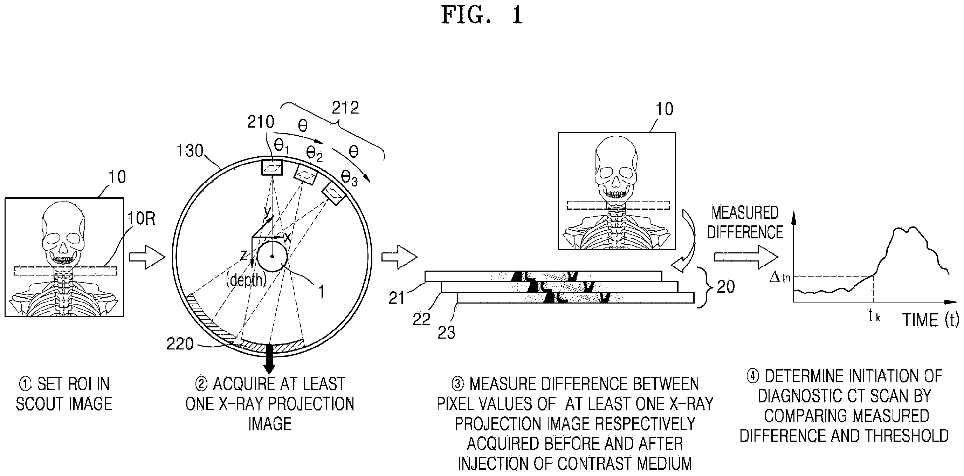

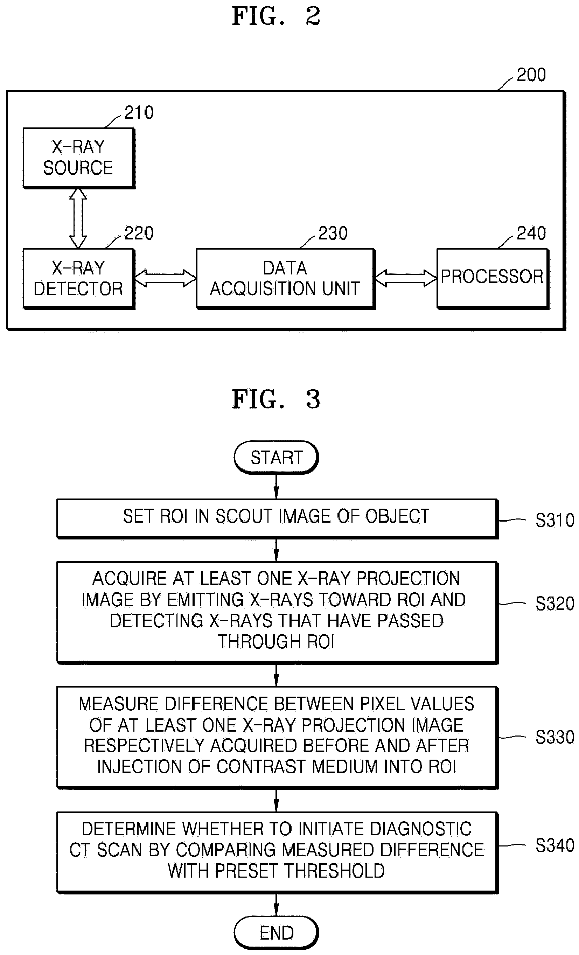

FIG. 1 is a conceptual diagram for explaining a method, performed by a CT apparatus 200, of determining a time point when a diagnostic CT scan is performed by injecting a contrast medium into an object 1, according to an embodiment. FIG. 2 is a block diagram illustrating components of a CT apparatus 200 according to an embodiment.

Referring to FIGS. 1 and 2, the CT apparatus 200 sets an ROI 10R in a scout image 10, acquires at least one X-ray projection image of the set ROI 10R, and measures a difference between pixel values of at least one X-ray projection image respectively obtained before and after injection of a contrast medium into the object 1 to determine whether to initiate a diagnostic CT scan based on the measured difference.

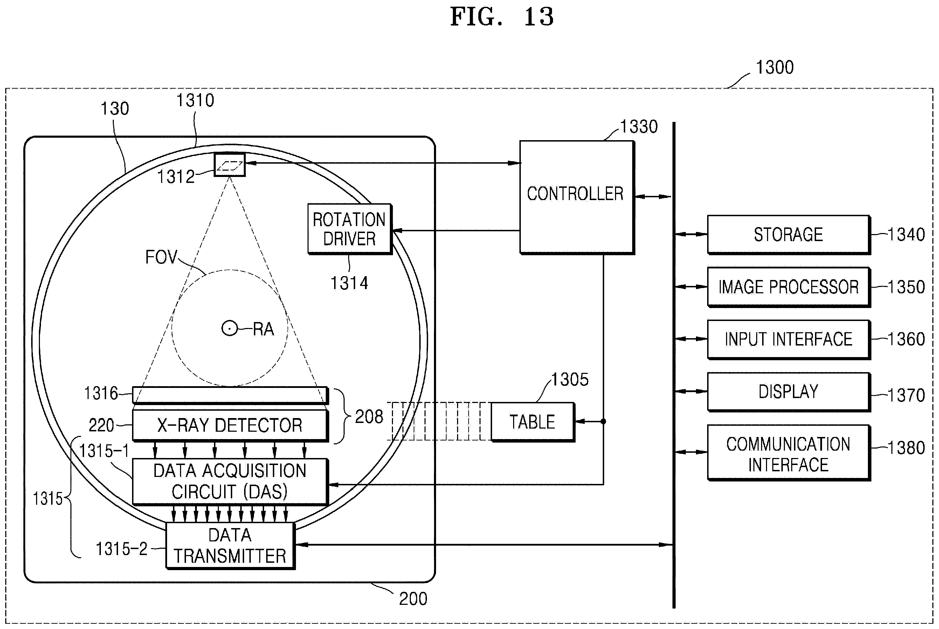

In detail, first, the CT apparatus 200 may capture the scout image 10 of the object 1 and set the ROI 10R in the captured scout image 10. The scout image 10 may be a preliminary image obtained by emitting X-rays toward a region to be scanned, i.e., a predetermined region of the object 1 including the ROI 10R, and detecting X-rays that have passed through the object 1. The scout image 10 may be an image captured for positioning an X-ray source 210 and an X-ray detector 220 with respect to the region to be scanned including the ROI 10R. The X-ray source 210 may be included in an X-ray generator 1312 (refer to FIG. 13).

The CT apparatus 200 may receive a user input of setting the ROI 10R in the scout image 10 and set the ROI 10R based on the received user input. In an embodiment, the CT apparatus 200 may set only a region to be observed by injecting a contrast medium into the object 1, e. g., a carotid artery or pulmonary artery, as the ROI 10R, or may set the ROI 10R to be a relatively wide portion including both a patient's shoulders and maxillary bones.

After setting the ROI 10R, the CT apparatus 200 may acquire at least one among X-ray projection images 20 respectively at at least one among angular positions 212 arranged around the object 1. According to an embodiment, the CT apparatus 200 may emit, via the X-ray source 210, X-rays toward the object 1 at a first angular position θ₁ and detect, by using the X-ray detector 220, the X-rays that have passed through the object 1 to acquire a first X-ray projection image 21. Then, after moving the X-ray source 210 by a preset angle θ on a rotating frame 130, the CT apparatus 200 may emit X-rays toward the object 1 at a second angular position θ₂ and detect the X-rays that have passed through the object 1 to acquire a second X-ray projection image 22. In the same manner, the CT apparatus 200 may acquire a third X-ray projection image 23 at a third angular position θ₃. Although FIG. 1 shows that the CT apparatus 200 acquires a total of 3 X-ray projection images including the first through third X-ray projection images 21 through 23 respectively at the first through third angular positions θ₁ through θ₃, this is merely an example, and the number of acquired X-ray projection images are not limited to 3, but may be any other appropriate natural number.

The CT apparatus 200 may inject a contrast medium into the ROI 10R of the object 1, such as a blood vessel, and measure a difference between pixel values of at least one X-ray projection image respectively obtained before and after injection of the contrast medium. According to an embodiment, the CT apparatus 200 may set, as a reference value, the sum of pixel values of at least one X-ray projection image, e.g., of a pre-contrast image, obtained before injection of a contrast medium into the ROI 10R. The CT apparatus 200 may sum up pixel values of at least one X-ray projection image respectively obtained at a plurality of time points following injection of the contrast medium into the ROI 10R and calculate a difference between a reference value and a result of summing up the pixel values obtained for each of the plurality of time points, as described below in detail with reference to FIG. 3.

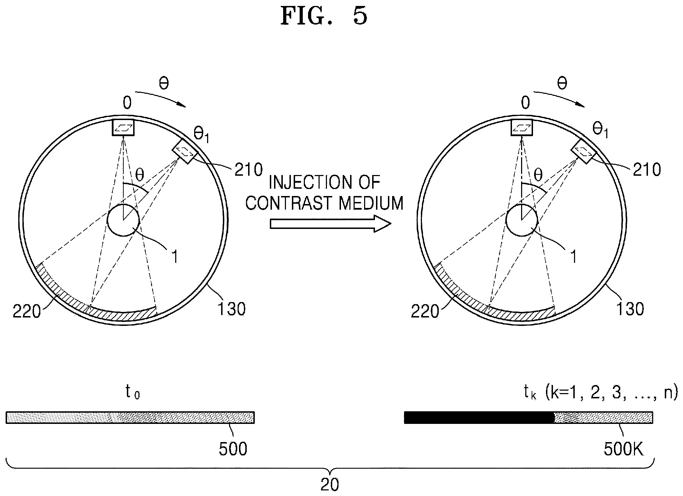

According to an embodiment, the CT apparatus 200 may set, as a reference value, a pixel value for the first X-ray projection image 21 obtained by emitting X-rays at a specific angular position, e.g., the first angular position θ₁, add up pixel values of an X-ray projection image acquired at the first angular position θ₁ at each of a plurality of time points following injection of a contrast medium, and calculate a difference between a result of adding up the pixel values and the reference value, as described in detail below with reference to FIG. 5.

The CT apparatus 200 may determine whether to initiate a diagnostic CT scan by comparing the calculated difference with a preset threshold. In this case, a diagnostic CT image may be reconstructed from a sinogram acquired by emitting X-rays via the X-ray source 210 that rotates 360 degrees around the object 1 and detecting the X-rays that have passed through the object 1 by using the X-ray detector 220.

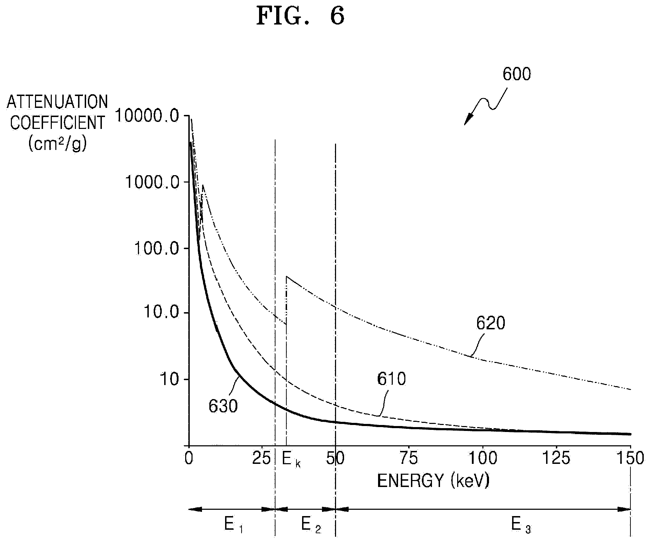

A difference between a reference value and the sum of pixel values of an X-ray projection image obtained at a time point after injection of a contrast medium into the ROI 10R of the object 1 may exceed a threshold Δₜₕ at a particular time point tₖ. The CT apparatus 200 may calculate a difference between the reference value and the sum of pixel values of an X-ray projection image obtained at each of a plurality of time points after injection of the contrast medium and determine the start of a diagnostic CT scan at the particular time point tₖ by tracking the calculated difference. The particular time point tₖ may be a time point when an attenuation coefficient of the contrast medium undergoes a rapid change, i.e., a time point when a contrast effect due to the contrast medium is maximized, as described in detail below with reference to FIG. 6.

According to an embodiment, the CT apparatus 200 may start a diagnostic CT scan after a lapse of a preset delay from the particular time point tₖ.

According to a bolus tracking technique, to obtain a high quality CT image exhibiting a maximized contrast effect, the X-rays are emitted at a time point when an attenuation coefficient of a contrast medium injected into an object reaches its maximum value. A related art bolus tracking method involves injecting a contrast medium into an ROI such as a carotid artery or a pulmonary artery, acquiring a pre-scan image by reconstructing an X-ray projection image generated by emitting X-rays in order to specify a time point when a contrast effect due to the contrast medium is maximized, and determining a time point for initiation of a diagnostic CT scan by setting an ROI in the acquired pre-scan image and measuring an (HU value in the ROI. In detail, the related art bolus tracking method is achieved by monitoring whether an HU value in the ROI is greater than a preset threshold and determining start of a diagnostic CT scan at a time point when the HU value is greater than the preset threshold. However, according to the related art bolus tracking method, a user suffers the inconvenience of having an ROI manually set in a pre-scan image, and when the ROI is set in a wrong region other than a region to be observed in the pre-scan image, a CT scan has to be performed again. Furthermore, it takes a long time to acquire a pre-scan image by reconstructing an X-ray projection image, and the user may miss a time point at which an attenuation coefficient for a contrast medium reaches its maximum value during reconstruction of the pre-scan image. In addition, since an X-ray source emits X-rays while rotating on a rotating frame through 360 degrees to acquire a pre-scan image, the amount of radiation to which a patient is exposed may increase excessively.

The CT apparatus 200 according to an embodiment is configured to determine a time point when a contrast effect due to a contrast medium is maximized by using a difference between pixel values of X-ray projection images respectively obtained before and after injection of the contrast medium into an ROI, reducing the amount of time required for reconstruction of a CT image. Since the reconstruction is accomplished in a short time, it is possible to solve the problem of missing the time point when a contrast effect due to the contrast medium is maximized.

Unlike a related art technique whereby a reconstruction process is performed after emitting X-rays during 360 degree rotation around the object 1 and detecting the X-rays that have passed through the object 1, the CT apparatus 200 according to an embodiment is configured to emit X-rays only at at least one of angular positions 212 around the object 1 and determine a time point at which a diagnostic CT scan is initiated by using at least one of X-ray projection images 20 acquired based on the emitted X-rays, thereby reducing the amount of X-ray radiation to which a patient is exposed.

Referring to FIG. 2, the CT apparatus 200 according to an embodiment may include an X-ray source 210, an X-ray detector 220, a data acquisition unit 230, and a processor 240. However, the CT apparatus 200 may further include a gantry 1310, a rotating frame 130, a table 1305, a display 1370, and a communication interface 1380, as shown in FIG. 13. In an embodiment, the CT apparatus 200 may further include a user input device or an input interface 1360 configured to receive a user input of setting an ROI in the scout image (10 of FIG. 1).

The X-ray source 210 may generate X-rays and emit the generated X-rays toward an object (e.g., a patient) while rotating on the rotating frame (130 of FIG. 1) positioned around the object. According to an embodiment, the X-ray source 210 may emit X-rays toward the object at the plurality of angular positions θ₁ through θ₃ while rotating about a rotation axis on the rotating frame 130 at preset angular intervals. For example, the X-ray source 210 may emit X-rays toward the object while rotating about a rotation axis at one-degree intervals. The X-ray source 210 may emit X-rays toward the object at angular positions corresponding to 1°, 2°, 3°, etc.

The X-ray detector 220 may detect the X-rays emitted by the X-ray source 210 toward the object. In an embodiment, the X-ray detector 220 may be formed as a photon counting detector (PCD) for detecting X-rays having a plurality of different energy bands. The X-ray detector 220 may detect X-rays emitted at a plurality of angles by which the X-ray source 210 rotates. For example, when the X-ray source 210 emits X-rays toward the object at angular positions of 1°, 2°, 3°, etc., the X-ray detector 220 may detect the X-rays emitted at the angular positions of 1°, 2°, 3°, etc.

The data acquisition unit 230 may acquire X-ray projection data output from the X-ray detector 220. The data acquisition unit 230 may include at least one amplifying circuit that may be used to amplify the X-ray projection data. The data acquisition unit 230 shown in FIG. 2 may be the same component as a data acquisition system (DAS) 1315-1 shown in FIG. 13. According to an embodiment, the data acquisition unit 230 may acquire at least one X-ray projection image from the X-ray detector 220 that detects X-rays emitted by the X-ray source 210 at at least one angular position as the X-ray source 210 rotates about an axis disposed at a center of the object. In an embodiment, the data acquisition unit 230 may form a sinogram by sequentially stacking at least one X-ray projection image respectively corresponding to at least one angular position.

The processor 240 may set an ROI in a scout image of the object, measure a difference between pixel values of at least one X-ray projection image respectively obtained before and after injection of a contrast medium into the ROI, and compare the measured difference with a preset threshold to determine whether to initiate a diagnostic CT scan. The processor 240 may be formed as a hardware unit having computational capabilities of converting pixel values of the at least one X-ray projection image acquired by the data acquisition unit 230 into numbers, adding up pixel values, and measuring a difference between pixel values before and after injection of a contrast medium. For example, the processor 240 may be constituted by at least one of a central processing unit (CPU), a microprocessor, and a graphic processing unit.

According to an embodiment, the X-ray source 210 may emit X-rays towards a predetermined region of the object including an ROI, and the X-ray detector 220 may detect the X-rays that have passed through the predetermined region of the object. The processor 240 may reconstruct a scout image from an X-ray projection image acquired based on the detected X-rays. In an embodiment, the user input device may receive a user input of setting an ROI in the scout image, such as a predetermined region including a blood vessel to be observed, and the processor 240 may set the ROI in the scout image based on the received user input.

According to an embodiment, the processor 240 may set, as a reference value, the sum of pixel values of at least one X-ray projection image obtained before injection of a contrast medium into an ROI, and calculate a difference between the reference value and the sum of pixel values of an X-ray projection image obtained at each of a plurality of time points after injection of the contrast medium into the ROI. The processor 240 may determine initiation of a diagnostic CT scan for examining the object at a time point when the calculated difference exceeds a preset threshold among the plurality of time points after injection of the contrast medium.

According to an embodiment, the processor 240 may set, as a reference value, the sum of pixel values of an X-ray projection image acquired at a particular angular position among X-ray projection images obtained before injection of a contrast medium into an ROI, and add up, for each of a plurality of time points, pixel values of an X-ray projection image acquired at the particular angular position among X-ray projection images acquired at the plurality of time points after injection of the contrast medium into the ROI. The processor 240 may then calculate a difference between the reference value and a result of adding up the pixel values obtained for each of the plurality of time points and determine initiation of a diagnostic CT scan at a time point when the calculated difference exceeds a preset threshold.

According to an embodiment, the X-ray detector 220 may be formed as a PCD, and may detect X-rays having an energy band in which an attenuation coefficient of a contrast medium reaches its maximum value among a plurality of energy bands. The data acquisition unit 230 may acquire at least one X-ray projection image based on the X-rays detected by the X-ray detector 220. The processor 240 may add up pixel values of the at least one X-ray projection image acquired by the data acquisition unit 230 and determine whether to initiate a diagnostic CT scan by calculating a difference between the sum of pixel values and a reference value.

According to an embodiment, the processor 240 may separate a contrast projection image to include only image data of those X-rays which have been detected when passing through only a contrast medium, from the acquired X-ray projection image. The processor 240 may respectively add up pixel values of the at least one X-ray projection image acquired at a plurality of time points following injection of the contrast medium and calculate a difference between a reference value and the sum of pixel values obtained for each of the plurality of time points. The processor 240 may determine the start of a diagnostic CT scan at a time point when the calculated difference exceeds a preset threshold.

FIG. 3 is a flowchart of a method, performed by the CT apparatus 200, of determining a time point when a diagnostic CT scan is performed by injecting a contrast medium into an object, according to an embodiment.

The CT apparatus 200 sets an ROI in a scout image of an object (operation S310). In an embodiment, the CT apparatus 200 may emit X-rays toward a predetermined region of the object including the ROI and detect the X-rays that have passed through the object to capture a scout image. The scout image may be an image captured for positioning the X-ray source 210 and the X-ray detector 220 with respect to a region to be scanned including the ROI.

The CT apparatus 200 may receive a user input of setting an ROI in a scout image and set the ROI in the scout image based on the received user input. According to an embodiment, the CT apparatus 200 may set as the ROI only a region to be observed by injecting a contrast medium into the object, e. g., a carotid artery or pulmonary artery, or may set the ROI to be a relatively wide portion including both a patient's shoulders and maxillary bones.

The CT apparatus 200 emits X-rays toward the ROI and detects the X-rays that have passed through the ROI to acquire at least one X-ray projection image (operation S320). According to an embodiment, the CT apparatus 200 may emit, via the X-ray source 210, X-rays at at least one angular position arranged around the object and detect, via the X-ray detector 220, the X-rays that have passed through the object. The CT apparatus 200 may generate at least one X-ray projection image by using the X-rays respectively detected at the at least one angular position. For example, the CT apparatus 200 may emit, via the X-ray source 210, X-rays toward the object at a first angular position and detect, by using the X-ray detector 220, the X-rays that have passed through the object to acquire a first X-ray projection image. Similarly, the CT apparatus 200 may emit, via the X-ray source 210, X-rays toward the object at a second angular position and detect, via the X-ray detector 220, the X-rays that have passed through the object to acquire a second X-ray projection image.

The CT apparatus 200 measures a difference between pixel values of at least one X-ray projection image respectively acquired before and after injection of a contrast medium into the ROI (operation S330). According to an embodiment, the CT apparatus 200 may set, as a reference value, the sum of pixel values of at least one X-ray projection image acquired before injection of the contrast medium into the ROI. The CT apparatus 200 sums up pixel values of at least one X-ray projection image respectively obtained at a plurality of time points following injection of the contrast medium into the ROI and calculate a difference between a reference value and the sum of pixel values obtained for each of the plurality of time points. For example, the CT apparatus 200 may emit, via the X-ray source 210, X-rays at at least one angular position at a first time point t₁, add up all pixel values of at least one X-ray projection image acquired by detecting the X-rays that have passed through the object, and calculate a difference between a first pixel value that is the resulting sum and a reference value. The CT apparatus 200 may add up all pixel values of at least one X-ray projection image acquired at a second time point t₂ and calculate a difference between a second pixel value that is the resulting sum and the reference value. In the same way, the CT apparatus 200 may add up all pixel values of at least one X-ray projection image acquired at a k-th time point tₖ and calculate a difference between a k-th pixel value that is the resulting sum and the reference value.

The CT apparatus 200 determines whether to initiate a diagnostic CT scan by comparing the measured difference with a preset threshold (operation S340). According to an embodiment, the CT apparatus 200 may compare a difference, which is calculated for each of a plurality of time points after injection of a contrast medium into the ROI, with a preset threshold. The CT apparatus 200 may determine to perform a diagnostic CT scan at a time point tₖ that is a time point when a calculated difference exceeds the preset threshold among the plurality of time points.

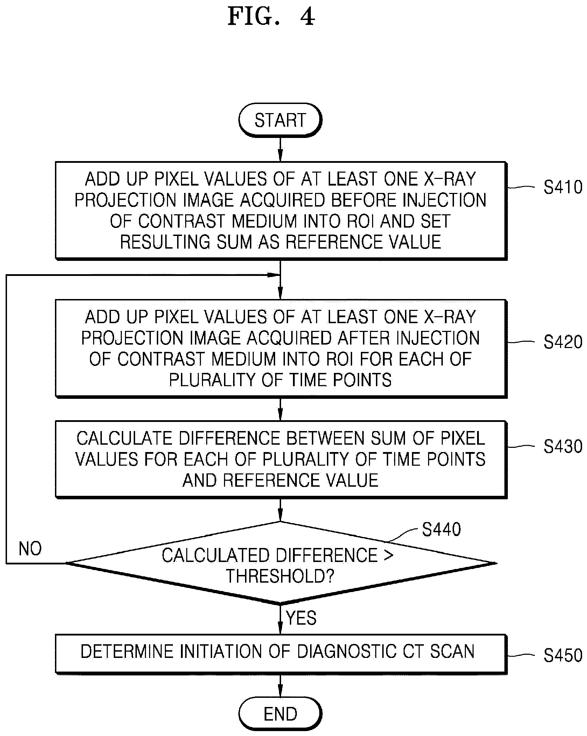

FIG. 4 is a flowchart of a method, performed by the CT apparatus 200, of determining a time point when a diagnostic CT scan is initiated by using X-ray projection images respectively obtained before and after injection of a contrast medium into an object, according to an embodiment.

The CT apparatus 200 adds up pixel values of at least one X-ray projection image acquired before injection of a contrast medium into an ROI and sets a result of adding up the pixel values as a reference value (operation S410). According to an embodiment, before injection of the contrast medium into the object, the CT apparatus 200 may emit, via the X-ray source 210, X-rays at at least one angular position arranged around the object and detect, via the X-ray detector 220, the X-rays that have passed through the object. The CT apparatus 200 may generate at least one X-ray projection image by using X-rays respectively detected at the at least one angular position. The CT apparatus 200 may sum up all pixel values of the at least one X-ray projection image acquired before injection of the contrast medium and set the resulting sum as a reference value. According to an embodiment, the CT apparatus 200 may sum up only pixel values of an X-ray projection image acquired, among X-ray projection images obtained before injection of the contrast medium, by emitting, via the X-ray source 210, X-rays toward the object at a particular angular position and detecting the X-rays that have passed through the object at the particular angular position, and set the resulting sum as a reference value. For example, the CT apparatus 200 may add up pixel values of a first X-ray projection image acquired by emitting X-rays at a first angular position θ₁ and set the resulting sum as a reference value.

The CT apparatus 200 adds up pixel values of at least one X-ray projection image acquired after injection of the contrast medium into the ROI for each of a plurality of time points (operation S420). According to an embodiment, the CT apparatus 200 may emit X-rays toward the object at a plurality of time points following injection of the contrast medium into the ROI and detect the X-rays that have passed through the object to acquire X-ray projection images. For example, the CT apparatus 200 may emit, via the X-ray source 210, X-rays at at least one angular position at a first time point t₁ after injection of the contrast medium into the ROI and detect the X-rays that have passed through the object to acquire at least one X-ray projection image. In the same manner, the CT apparatus 200 may emit, via the X-ray source 210, X-rays toward the object at a second time point t₂ after the first time point t₁ at the same angular position as for the first time point t₁ and detect the X-rays that have passed through the object to acquire at least one X-ray projection image.

The CT apparatus 200 calculates a difference between the sum of pixel values for each of the plurality of time points and the reference value (operation S430). According to an embodiment, the CT apparatus 200 may calculate a first pixel value by adding up all pixel values of at least one X-ray projection image acquired at a first time point t₁ and determine a difference between the first pixel value and the reference value.

The CT apparatus 200 compares the calculated difference with a preset threshold (operation S440). According to an embodiment, the CT apparatus 200 may track whether a difference calculated for each of a plurality of time points exceeds a preset threshold. For example, the CT apparatus 200 may determine whether a difference between the reference value and a first pixel value, which is calculated at the first time point t₁, exceeds a threshold.

When the calculated difference does not exceed the preset threshold (no in operation S440), the CT apparatus 200 returns to operation S420 of acquiring at least one X-ray projection image at a second time point t₂, calculating a second pixel value by adding up pixel values of the acquired at least one X-ray projection image, and determining a difference between the second pixel value and the reference value. The CT apparatus 200 may determine whether a difference between the reference value and a second pixel value calculated at the at the second time point t₂ exceeds the threshold in operation S440. Until reaching a time point when the calculated difference exceeds the preset threshold, the CT apparatus 200 may continue to acquire an X-ray projection image at a plurality of time points, calculate a difference between the sum of pixel values of the acquired X-ray projection image and a reference value, and compare the calculated difference with the preset threshold.

When the calculated difference exceeds the preset threshold (yes in operation S440), the CT apparatus 200 determines initiation of a diagnostic CT scan (operation S450). In operation S450, a diagnostic CT image may be acquired as an image reconstructed from a sinogram acquired by emitting X-rays as the X-ray source 210 rotates 360 degrees around the object and detecting, by using the X-ray detector 220, the X-rays that have passed through the object.

The CT apparatus 200 may initiate a diagnostic CT scan immediately after a time point tₖ when the calculated difference exceeds the preset threshold in operation S440, but embodiments are not limited thereto. In an embodiment, the CT apparatus 200 may determine initiation of a diagnostic CT scan after a lapse of a preset delay from the time point tₖ when the calculated difference exceeds the preset threshold.

FIG. 5 is a diagram for explaining a method, performed by the CT apparatus 200, of determining a time point when a diagnostic CT scan is initiated by using X-ray projection images 20 including an X-ray projection image 500 acquired at time point t₀ and an X-ray projection image 500ₖ acquired at time point tₖ, by emitting X-rays toward an object 1 at a particular angular position, according to an embodiment. Although FIG. 5 shows only two X-ray projection images acquired at two time points t₀ and tₖ, a different number of X-ray projection images may be acquired at the time points between t₀ and tₖ or additional X-ray projection images may be acquired after the time point tₖ.

Referring to FIG. 5, the CT apparatus 200 may emit, via the X-ray source 210, X-rays toward the object 1 before a time point t₀ that is before injection of a contrast medium into an ROI of the object 1, and detect, via the X-ray detector 220, the X-rays that have passed through the object 1 to acquire an X-ray projection image 500. The X-ray source 210 may emit X-rays toward the object 1 at a first angular position θ₁ on a rotating frame 130 positioned around the object, and the X-ray detector 220 positioned opposite the first angular position θ₁ may detect the X-rays that have passed through the object 1 to acquire the X-ray projection image 500. The first angular position θ₁ may be a position obtained as the X-ray source 210 rotates from a zero-degree position on the rotating frame 130 by a preset angle θ.

The CT apparatus 200 may add up pixel values of the X-ray projection image 500 acquired at the time point t₀ that is before injection of the contrast medium into the ROI and set the resulting sum as a reference value.

The CT apparatus 200 may inject the contrast medium into the ROI of the object 1 and acquire X-ray projection images at a plurality of time points following injection of the contrast medium. According to an embodiment, the CT apparatus 200 may move the X-ray source 210 to the first angular position θ₁ at a particular time point tₖ following the injection of the contrast medium into the ROI and then detect, by using the X-ray detector 220 positioned opposite the first angular position θ₁, X-rays that have passed through the ROI of the object 1 to acquire an X-ray projection image 500ₖ.

The CT apparatus 200 may add up pixel values of the X-ray projection image 500k acquired at the particular time point tₖ following injection of the contrast medium into the ROI and calculate a difference between a result of adding up the pixel values and the reference value. The CT apparatus 200 may determine to perform a diagnostic CT scan at a time point when the calculated difference exceeds a preset threshold.

In the embodiment shown in FIG. 5, the CT apparatus 200 may emit X-rays at the same angular position, i.e., the first angular position θ₁ at both the time points t₀ and tₖ that are respectively before and after injection of the contrast medium into the ROI of the object 1, and determine a time point when the a diagnostic CT scan is initiated based on a difference between pixel values of the X-ray projection images 500 and 500k acquired by detecting the X-rays that have passed through the object 1.

FIG. 6 is a graph 600 of attenuation coefficients of a bone of an object, water, and contrast medium with respect to energy.

Referring to the graph 600 of FIG. 6, attenuation coefficients 610 and 630 of a bone and water tend to decrease gradually as energy increases. However, an attenuation coefficient 620 of a contrast medium undergoes a rapid change in a particular energy band among a plurality of energy bands E₁ through E₃. In other words, the attenuation coefficient 620 of the contrast medium may increase or decrease rapidly in a particular energy band when energy increases. For example, the attenuation coefficient 620 of the contrast medium may increase rapidly in a second energy band E₂ and decrease again as an energy band becomes higher. An energy in an energy band in which the attenuation coefficient 620 of the contrast medium undergoes a rapid change may be defined as a k-edge energy Eₖ, and the attenuation coefficient 620 of the contrast medium may increase rapidly at k-edge energy Eₖ.

While the contrast medium in FIG. 6 may be iodine, the type of contrast medium used is not limited thereto. In an embodiment, the contrast medium injected into the ROI may be gadolinium. When the contrast medium is iodine, iodine has a k-edge at which an attenuation coefficient undergoes a sudden change at about 33 kilo-electronvolts (keV) contained in the second energy band E₂. In other words, iodine has a k-edge energy Eₖ of about 33 keV. When the contrast medium is gadolinium, gadolinium has a k-edge energy Eₖ at 30.2 keV at which an attenuation coefficient undergoes a rapid change. In an embodiment, the second energy band E₂ may be in a range of between 30 keV and 50 keV. However, the range of the second energy band E₂ is not limited thereto.

The X-ray detector 220 included in the CT apparatus 200 may be PCD for detecting X-rays having a plurality of different energy bands. The CT apparatus 200 may detect, via a PCD, X-rays having different energy bands and which have passed through an ROI of an object and acquire a plurality of X-ray projection images by using the detected X-rays.

The CT apparatus 200 may determine a time point when a diagnostic CT scan is initiated by using an X-ray projection image generated, among a plurality of X-ray projection images, by using X-rays having an energy corresponding to a k-edge that is in an energy band in which an attenuation coefficient of a contrast medium injected into an ROI increases rapidly. According to an embodiment, when an iodine-based contrast medium is injected into the ROI, the CT apparatus 200 may acquire a second X-ray projection image by using X-rays having the second energy band E₂ including 33 keV that is a k-edge energy of iodine. The CT apparatus 200 may add up pixel values of an X-ray projection image acquired before injection of the contrast medium into the object to set the resulting sum as a reference value, and add up pixel values of the second X-ray projection image acquired after injection of the contrast medium to calculate a difference between a result of adding up the pixel values and the reference value for each of a plurality of time points. The CT apparatus 200 may then determine initiation of a diagnostic CT scan at a time point when the difference between the result of adding up the pixel values and the reference value exceeds a preset threshold.

Since the degree of attenuation due to a contrast medium increases with an increase in an attenuation coefficient of the contrast medium, pixel values of an ROI, i.e., a blood vessel, in a CT image may increase. In other words, since the blood vessel appears white on the CT image, it is possible to achieve a maximum contrast effect between the blood vessel and a bone or water that appears black or gray on the CT image. To maximize a contrast effect due to the contrast medium in a CT image, a CT scan needs to be performed in an energy band (e. g., the second energy band E₂) in which an attenuation coefficient of the contrast medium increases to the greatest extent. According to an embodiment, the CT apparatus 200 may detect, via a PCD, X-rays having a plurality of different energy bands and determine a time point for initiation of a diagnostic CT scan by using pixel values of an X-ray projection image acquired in an energy band (e.g., the second energy band E₂) in which the attenuation coefficient of the contrast medium increases to the greatest extent, thereby allowing acquisition of a high quality CT image exhibiting a maximum contrast effect due to the contrast medium.

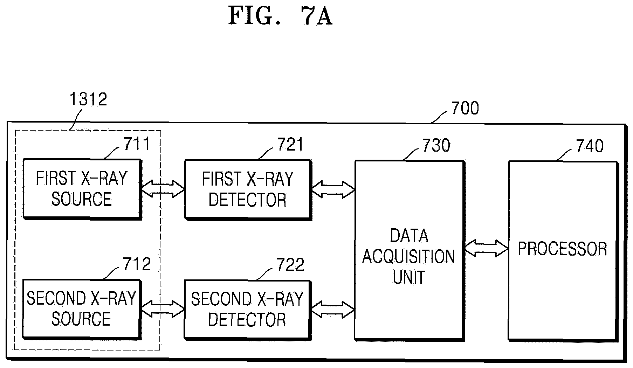

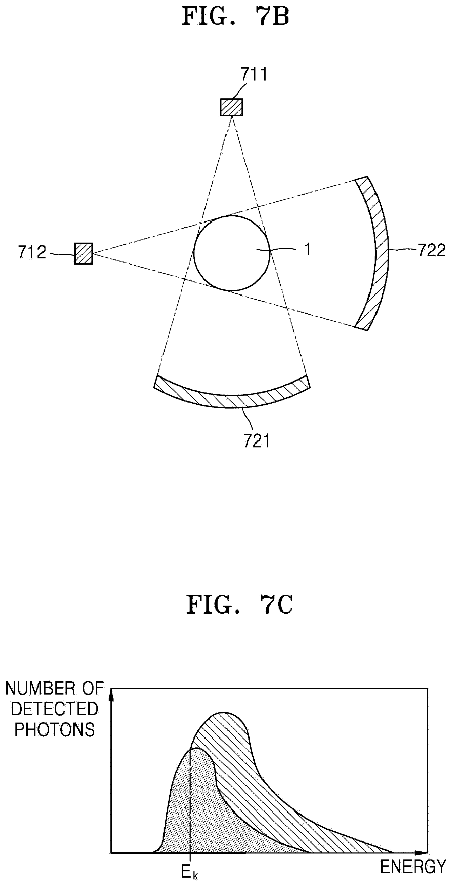

FIG. 7A is a block diagram showing components of a CT apparatus 700 according to an embodiment. FIG. 7B is a diagram for explaining a method, performed by the CT apparatus 700 of FIG. 7A, of performing a CT scan by emitting X-rays toward an object 1 at different tube voltages, and FIG. 7C is a graph of the number of photons contained in X-rays, which are emitted by the CT apparatus 700 of FIG. 7A, pass through an object, and are detected thereby, with respect to an energy band. The CT apparatus 700 may correspond to the CT apparatus 200.

Referring to FIG. 7A, the CT apparatus 700 may include the X-ray generator 1312 including first and second X-ray sources 711 and 712, first and second X-ray detectors 721 and 722, a data acquisition unit 730, and a processor 740. The data acquisition unit 730 and the processor 740 may be the same components as the data acquisition unit 230 and the processor 240 described with reference to FIG. 2, respectively. The descriptions that are provided above with respect to FIG. 2 will be omitted here.

The first and second X-ray sources 711 and 712 may respectively emit X-rays toward an object at different tube voltages (kilovoltage peak; kVp). For example, the first and second X-ray sources 711 and 712 may emit X-rays toward the object at 80 kVp and 140 kVp, respectively. Referring to 7B, the first X-ray source 711 may emit X-rays toward the object 1 at a first position at a tube voltage of 80 kVp, and the second X-ray source 712 may emit X-rays toward the object 1 at a second position at a tube voltage of 140 kVp.

The first and second X-ray detectors 721 and 722 may respectively detect the X-rays that are respectively emitted by the first and second X-ray sources 711 and 712 and pass through the object. Referring to FIG. 7B, the first X-ray detector 721 may detect the X-rays that are emitted by the first X-ray source 711 and pass through the object 1. The second X-ray detector 722 may detect X-rays that are emitted by the second X-ray source 712 and pass through the object. The number of photons in X-rays respectively detected by the first and second X-ray detectors 721 and 722 may vary according to an energy band, as described in detail below with reference to FIG. 7C.

The data acquisition unit 730 may generate a plurality of X-ray projection images based on X-rays respectively detected by the first and second X-ray detectors 721 and 722. Referring to FIG. 7C, X-rays emitted by the first X-ray source 711 at a tube voltage of 80 kVp may have a lower energy band than X-rays emitted by the second X-ray source 712 at a tube voltage of 140 kVp. The data acquisition unit 730 may generate a first X-ray projection image by using X-rays having a first energy band that is a low energy band, and produce a second X-ray projection image by using X-rays having a second energy band that is a relatively high energy band.

The processor 740 may measure a difference between pixel values of an X-ray projection image acquired before injection of a contrast medium, among the first and second X-ray projection images, and an X-ray projection image acquired by detecting X-rays having an energy corresponding to a k-edge energy Eₖ that is in an energy band in which the degree of attenuation of the contrast medium injected into the object 1 is at a maximum. For example, when the contrast medium injected into the object 1 is iodine, since iodine has a k-edge energy Eₖ at about 33 keV, the processor 740 may add up pixel values of the first X-ray projection image acquired by detecting the X-rays emitted at a tube voltage of 80 kVp. The processor 740 may add up pixel values of an X-ray projection image generated by the data acquisition unit 730 before injection of the contrast medium into the object 1 and set the resulting sum as a reference value. The processor 740 may add up pixel values of the first X-ray projection image respectively acquired at a plurality of time points following injection of the contrast medium into the object 1 and calculate a difference between a result of adding up the pixel values and the reference value for each of the plurality of time points.

According to an embodiment, the processor 740 may measure a time point when the calculated difference exceeds a preset threshold and determine initiation of a diagnostic CT scan at the measured time point.

The CT apparatus 700 according to the embodiment shown in FIGS. 7A through 7C may emit X-rays at different tube voltages via the plurality of X-ray sources (the first and second X-ray sources 711 and 712), detect the X-rays having different energy bands via the plurality of X-ray detectors (the first and second X-ray detectors 721 and 722), and acquire a plurality of X-ray projection images by using the detected X-rays. The CT apparatus 700 may determine a time point when the diagnostic CT scan is initiated by using pixel values of an X-ray projection image acquired, among the plurality of X-ray projection mages, by using X-rays having an energy band corresponding to a k-edge energy Eₖ of the contrast medium.

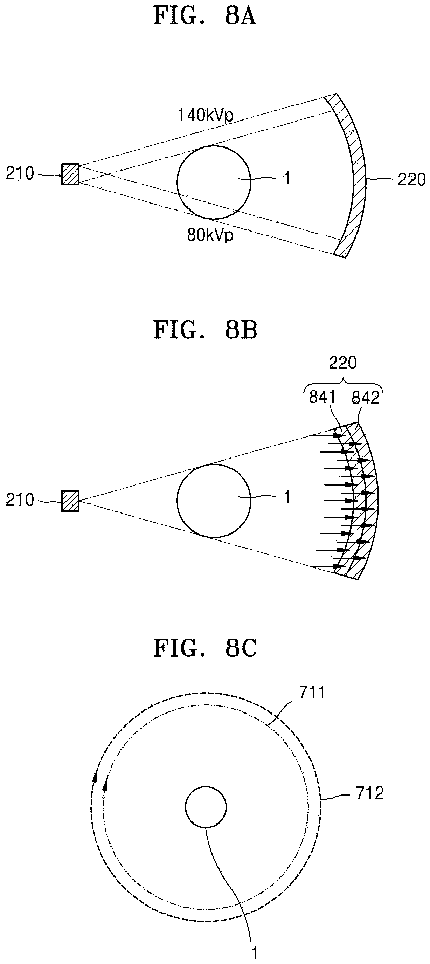

FIGS. 8A through 8C are conceptual diagrams for explaining methods, performed by CT apparatus 200, of obtaining a plurality of X-ray projection images by emitting X-rays having a plurality of different energy bands toward an object, according to embodiments.

Referring to FIG. 8A, the CT apparatus 200 may emit X-rays toward an object 1 by applying different tube voltages to an X-ray source 210. In an embodiment, the CT apparatus 200 may apply tube voltages of 80 kVp and 140 kVp to the X-ray source 210 and emit X-rays toward the object at 80 kVp and 140 kVp. An X-ray detector 220 may detect X-rays that are emitted at the different tube voltages and penetrate the object 1. According to an embodiment, the X-ray detector 220 may detect X-rays having different energy bands.

The CT apparatus 200 may acquire at least one X-ray projection image by using, among X-rays having different energy bands detected by the X-ray detector 220, X-rays having an energy corresponding to a k-edge that is in an energy band in which an attenuation coefficient of a contrast medium injected into the object 1 undergoes a largest variation. According to an embodiment, the CT apparatus 200 may add up pixel values of the acquired at least one X-ray projection image and determine a time point when a diagnostic CT scan is initiated based on a difference between a result of adding up the pixel values and a reference value which is a sum of pixel values for an X-ray projection image acquired before injection of the contrast medium.

Referring to FIG. 8B, the CT apparatus 200 may include an X-ray source 210 and an X-ray detector 220. The X-ray detector 220 may include first and second X-ray detection layers 841 and 842. The first and second X-ray detection layers 841 and 842 may respectively detect X-rays having different energy bands. In an embodiment, the first X-ray detection layer 841 may detect X-rays having a lower energy band than X-rays detected by the second X-ray detection layer 842.

The CT apparatus 200 may acquire at least one X-ray projection image by using the X-rays respectively detected by the first and second X-ray detection layers 841 and 842. The CT apparatus 200 may acquire at least one X-ray projection image via an X-ray detector that has detected, among the X-rays respectively detected by the first and second X-ray detection layers 841 and 842, X-rays having an energy band corresponding to a k-edge of a contrast medium injected into an object 1. According to an embodiment, the CT apparatus 200 may add up pixel values of the acquired at least one X-ray projection image and determine a time point when a diagnostic CT scan is initiated based on a difference between a result of adding up the pixel values and a pixel value for an X-ray projection image acquired before injection of the contrast medium.

Referring to FIG. 8C, the CT apparatus 200 may include the X-ray generator 1312 including a plurality of X-ray sources, i.e., first and second X-ray sources 711 and 712. The first and second X-ray sources 711 and 712 may respectively emit X-rays toward an object 1 at different tube voltages. The first and second X-ray sources 711 and 712 may rotate on a rotating frame within a gantry. The first and second X-ray sources 711 and 712 may alternately emit X-rays toward the object 1 while rotating on the rotating frame 130.

The CT apparatus 200 may detect X-rays having different energy bands that are respectively emitted by the first and second X-ray sources 711 and 712 and pass through the object 1, by a single X-ray detector or two X-ray detectors. The CT apparatus 200 may then acquire at least one X-ray projection image by using the X-rays having different energy bands.

According to an embodiment, the CT apparatus 200 may acquire at least one first X-ray projection image by detecting X-rays emitted by the first X-ray source 711 and at least one second X-ray projection image by detecting X-rays emitted by the second X-ray source 712. The CT apparatus 200 may also determine a time point when a diagnostic CT scan is initiated based on pixel values of an X-ray projection image generated, among the at least one first X-ray projection image and the at least one second X-ray projection image, by using X-rays having an energy band corresponding to a k-edge of a contrast medium injected into the object 1.

According to an embodiment, the CT apparatus 200 may add up pixel values of the X-ray projection image generated using the X-rays having an energy band corresponding to the k-edge of the contrast medium at each of a plurality of time points, measure a difference between a result of adding up the pixel values and a pixel value for an X-ray projection image acquired before injection of the contrast medium, and determine initiation of a diagnostic CT scan at a time point when the calculated difference exceeds a preset threshold.

In general, iodine is used as a contrast medium during a CT scan, and has a k-edge energy Eₖ of 33 keV. Thus, by detecting, among X-rays having a plurality of different energy bands, X-rays having an energy band containing 33 keV and generating an X-ray projection image based on the detected X-rays, it is possible to maximize a contrast effect between a blood vessel that is an ROI of the object 1 and the remaining regions other than the ROI, such as a bone, skin, water, etc.

The CT apparatus 200 according to embodiments described with reference to FIGS. 8A through 8C may detect X-rays having different energy bands and determine a time point for initiation of a diagnostic CT scan by using pixel values of an X-ray projection image acquired using, among the detected X-rays, X-rays having an energy band in which an attenuation coefficient of a contrast medium injected into the object 1 increases rapidly, e.g., X-rays having an energy corresponding to 33 keV when the contrast medium is iodine or X-rays having an energy corresponding to a different keV value when the contrast medium is other than iodine.

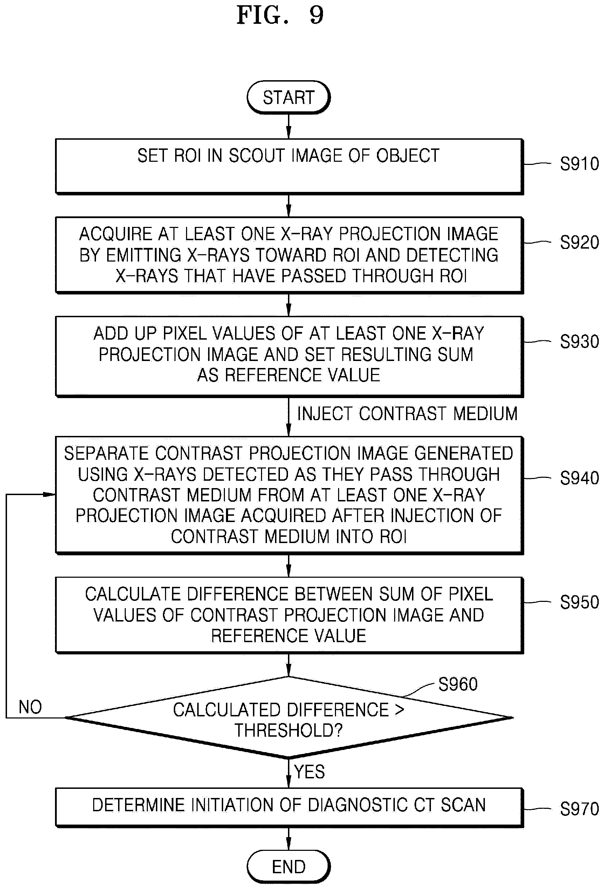

FIG. 9 is a flowchart of a method, performed by a CT apparatus, of separating a contrast projection image from an X-ray projection image of an object and determining whether to initiate a diagnostic CT scan by using the separated contrast projection image, according to an embodiment.

The CT apparatus sets an ROI in a scout image of an object (operation S910). According to an embodiment, the CT apparatus may emit X-rays toward a predetermined region of the object including the ROI and detect the X-rays that have passed through the object to capture a scout image. The scout image may be an image captured for positioning an X-ray source and an X-ray detector with respect to a region to be scanned including the ROI.

The CT apparatus emits X-rays toward the ROI and detects the X-rays that have passed through the ROI to acquire at least one X-ray projection image (operation S920). In an embodiment, the CT apparatus may emit, via the X-ray source, X-rays at at least one angular position arranged around the object, detect, by using the X-ray detector, the X-rays that have passed through the object, and generate at least one X-ray projection image by using the detected X-rays.

The CT apparatus adds up pixel values of the at least one X-ray projection image and sets the resulting sum as a reference value (operation S930). According to an embodiment, before injection of a contrast medium into the object, the CT apparatus may emit, via the X-ray source, X-rays at at least one angular position arranged around the object and detect, by using the X-ray detector, the X-rays that have passed through the object.

The CT apparatus may sum up all pixel values of the at least one X-ray projection image acquired before injection of the contrast medium and set the resulting sum as a reference value. According to an embodiment, the CT apparatus may sum up only pixel values of an X-ray projection image acquired, among the at least one X-ray projection image obtained before injection of the contrast medium, by emitting, via the X-ray source, X-rays toward the object at a particular angular position and detecting the X-rays that have passed through the object at the particular angular position, and set the resulting sum as a reference value.

The CT apparatus separates a contrast projection image from X-ray projection images acquired after injection of the contrast medium into the ROI (operation S940). The contrast projection image may mean a projection image generated using X-rays detected when passing through only the ROI of the object into which the contrast medium is injected.

According to an embodiment, the CT apparatus may separate only a contrast projection image from at least one X-ray projection image acquired at a plurality of time points following injection of the contrast medium into the ROI. For example, when a CT scan of a patient's neck is performed by injecting a contrast medium into a blood vessel including a carotid artery in order to observe the carotid artery, the CT apparatus may separate a contrast projection image that is an X-ray projection image of the carotid artery from an X-ray projection image acquired by emitting X-rays toward the patient's neck.

The CT apparatus calculates a difference between the sum of pixel values of the contrast projection image and the reference value (operation S950). According to an embodiment, the CT apparatus may add up pixel values of a contrast projection image separated at each of a plurality of time points following injection of the contrast into the ROI. The CT apparatus may calculate a difference between a result of adding up the pixel values and the reference value for each of the plurality of time points.

For example, the CT apparatus may add up pixel values of a contrast projection image at a first time point t₁, and calculate a difference between a first pixel value that is the resulting sum and the reference value. The CT apparatus may add up pixel values of a contrast projection image at a second time point t₂ and calculate a difference between a second pixel value that is the resulting sum and the reference value. Similarly, the CT apparatus may add up all pixel values of a contrast projection image at a k-th time point tₖ and calculate a difference between a k-th pixel value that is the resulting sum and the reference value.

The CT apparatus compares the calculated difference with a preset threshold (operation S960). According to an embodiment, the CT apparatus may track whether a difference calculated for each of a plurality of time points exceeds a preset threshold. For example, the CT apparatus may determine at a first time point t₁ whether a difference between the reference value and a first pixel value that is the sum of pixel values of a contrast projection image calculated at the first time point t₁ exceeds the preset threshold. Similarly, the CT apparatus may determine at a second time point t₂ whether a difference between the reference value and a second pixel value that is the sum of pixel values of a contrast projection image calculated at the second time point t₂ exceeds the preset threshold.

When the calculated difference does not exceed the preset threshold (no in operation S960), the CT apparatus returns to operation S940 of separating a contrast projection image at a third time point t₃ and calculating a third pixel value by adding up pixel values of the contrast projection image at the third time point t₃. Until reaching a time point when the calculated difference exceeds the preset threshold, the CT apparatus may continue to separate a contrast projection image at a plurality of time points, calculate a difference between the sum of pixel values of the contrast projection image and the reference value, and compare the calculated difference with the preset threshold.

When the calculated difference exceeds the preset threshold (yes in operation S960), the CT apparatus determines initiation of a diagnostic CT scan (operation S970). In operation S970, a diagnostic CT image may be an image reconstructed from a sinogram acquired by emitting X-rays as an X-ray source rotates around the object through 360 degrees and detecting, via an X-ray detector, the X-rays that have passed through the object.

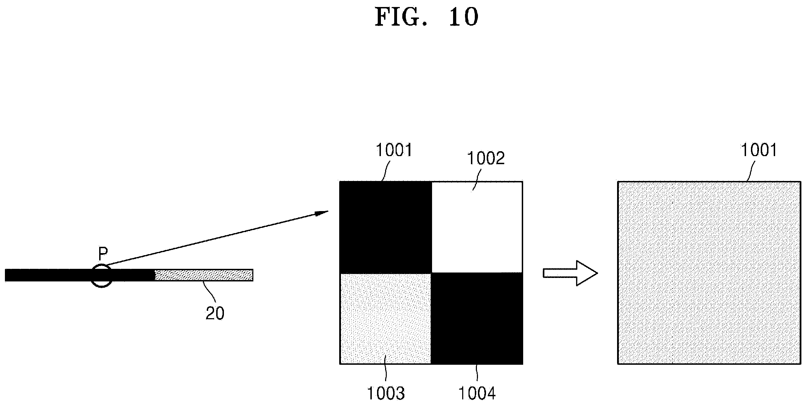

FIG. 10 illustrates a method, performed by a CT apparatus, of binning pixels in an X-ray projection image such that noise in the X-ray projection image is reduced, according to an embodiment.

Referring to FIG. 10, the CT apparatus may change values of a preset number of adjacent pixels 1001, 1002, 1003, and 1004 among pixels of an X-ray projection image of an ROI of an object. For example, the CT apparatus may calculate an average of values of four pixels 1001 through 1004 included in a pixel group P of the X-ray projection image and replace all the values of the four pixels 1001 through 1004 with the calculated average.

When at least one X-ray projection image is generated by emitting X-rays at only one or some of angular positions θ₁, θ₂, θ₃, ... on a rotating frame instead of entire 360 degrees, noise occurs in the X-ray projection image as compared to that acquired in a complete 360-degree sinogram. The CT apparatus of FIG. 10 according to an embodiment may calculate an average of values of the preset number of adjacent pixels 1001 through 1004 and replace the values of the adjacent pixels 1001 through 1004 with the calculated average, thereby reducing noise in the X-ray projection image.

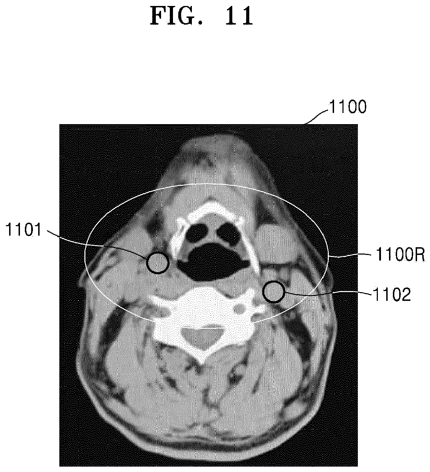

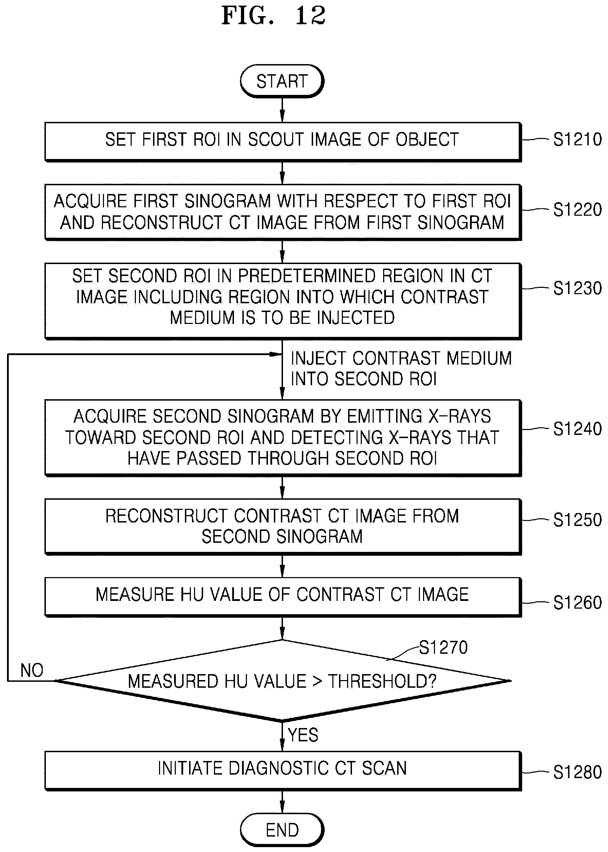

FIG. 11 is a diagram for explaining a method of setting an ROI in a scout image of an object by using a CT apparatus, according to an embodiment, and FIG. 12 is a flowchart of a method, performed by a CT apparatus, of performing a diagnostic CT scan on an object, according to an embodiment.

Referring to FIG. 12, the CT apparatus sets a first ROI in a scout image of an object (operation S1210). According to an embodiment, the CT apparatus may emit X-rays toward a region of an object to be scanned, e.g., a predetermined region including a patient's neck, chest, brain, etc., and detect the X-rays that have passed through the object to capture a scout image. The scout image may be an image captured for positioning an X-ray source and an X-ray detector with respect to a region of the patient to be scanned.

The CT apparatus acquires a first sinogram with respect to the first ROI and reconstructs a CT image from the first sinogram (operation S1220). According to an embodiment, the CT apparatus may rotate an X-ray source 360 degrees on a rotating frame positioned around the object and emit X-rays toward the first ROI via the X-ray source. The CT apparatus may detect, by using an X-ray detector, the X-rays that have passed through the first ROI to acquire a first sinogram that is a set of a plurality of X-ray projection images corresponding to angular positions from 0 to 360 degrees. The CT apparatus may then reconstruct a CT image from the first sinogram by using a filtered back-projection (FBP) algorithm, etc.

The CT apparatus sets a second ROI in a predetermined region on a CT image, including a region into which a contrast medium is to be injected (operation S1230). Referring to FIG. 11, the CT apparatus may set a second ROI 1100R in a CT image 1100 displayed on a screen of a display 1370 (refer to FIG. 13). For example, during a CT scan of a patient's neck, the CT apparatus may set the second ROI 1100R in a relatively wide region including blood vessels 1101 and 1102 into which a contrast medium is to be injected without receiving a user input of specifying the blood vessels 1101 and 1102. When the CT scan is performed by injecting the contrast medium into the blood vessels 1101 and 1102 of the patient, a user of the CT apparatus described with reference to FIGS. 11 and 12 does not need to individually specify the blood vessels 1101 and 1102. Thus, user's convenience may be improved.

The CT apparatus emits X-rays toward the second ROI (1100R of FIG. 11) and detects the X-rays that have passed through the second ROI 1100R to acquire a second sinogram (operation S1240). According to an embodiment, the CT apparatus may emit, via the X-ray source, X-rays toward the second ROI 1100R of the object at angular positions from 0 to 360 degrees and detect the X-rays that have passed through the second ROI 1100R to acquire a second sinogram that is a set of plurality of X-ray projection images.

The CT apparatus reconstructs a contrast CT image from the second sinogram (operation S1250).

The CT apparatus measures an HU value in the contrast CT image (operation S1260). According to an embodiment, the CT apparatus may acquire a sinogram at a time point following injection of the contrast medium into the second ROI, e.g., blood vessels, and reconstruct a contrast image from the acquired sinogram. The CT apparatus may also measure an HU value of the contrast CT image.

The CT apparatus compares the measured HU value with a preset threshold (operation S1270). According to an embodiment, the CT apparatus may respectively compare HU values of first through k-th CT images with a preset threshold for a plurality of time points t₁ through tₖ. When the measured HU value does not exceed the preset threshold (no in operation S1270), the CT apparatus repeats operation S1240 of emitting X-rays toward the second ROI and detecting the X-rays that have passed through the second ROI to acquire a second sinogram, operation S1250 of generating a contrast CT image, and operation S1250 of measuring an HU value of the contrast CT image.

According to an embodiment, the CT apparatus may repeat operations S1240 through S1270 at a plurality of time points following injection of the contrast medium into the second ROI. For example, the CT apparatus may acquire a sinogram with respect to the second ROI at a first time point t₁ after injection of the contrast medium into the second ROI and reconstruct a first contrast CT image from the acquired sinogram. The CT apparatus may then compare an HU value of the first contrast CT image with the preset threshold (operation S1270) and, when the HU value of the first contrast CT image does not exceed the preset threshold (no in operation S1270), return to operation S1240 to generate a second contrast CT image. When an HU value of a k-th CT image at a k-th time point tₖ exceeds the preset threshold (yes in operation S1270), the CT apparatus determines initiation of a diagnostic CT scan (operation S1280).

According to a related art bolus tracking method, when an ROI is set in a pre-scan image, the user suffers the inconvenience of having to directly select a blood vessel such as carotid artery into which a contrast medium is to be injected for observation. When the blood vessel such as the carotid artery is not set correctly as the ROI, a CT scan has to be performed again.

According to the embodiment described with reference to FIGS. 11 and 12, the CT apparatus is configured to detect X-rays having a plurality of different energy bands via a PCD, generate an X-ray projection image by using, among the detected X-rays, X-rays having an energy region in which the degree of attenuation due to the contrast medium is maximized, and reconstruct a CT image from the X-ray projection image, thereby eliminating the need for directly setting the blood vessels (1101 and 1102 of FIG. 11) as an ROI. In other words, the CT apparatus according to the embodiment may set the second ROI 1100R to be a relatively wide region including not only the blood vessels 1101 and 1102 but also the surrounding region, thereby improving user convenience. Furthermore, it is possible to prevent repetition of a CT scan due to erroneous setting of an ROI, thereby reducing the amount of patient's radiation exposure.

FIG. 13 illustrates a structure of a CT system 1300 according to an embodiment.

The CT system 1300 may include a gantry 1310, a table 1305, a controller 1330, a storage 1340, an image processor 1350, an input interface 1360, a display 1370, and a communication interface 1380.

The gantry 1310 may include a rotating frame 130, an X-ray generator 1312, an X-ray detector 220, a rotation driver 1314, and a readout device 1315.

The rotating frame 130 may receive a driving signal from the rotation driver 1314 and rotate around a rotation axis (RA).

An anti-scatter grid 1316 may be disposed between an object and the X-ray detector 220 and may transmit most of primary radiation and attenuate scattered radiation. The object may be positioned on the table 1305 which may move, tilt, or rotate during a CT scan.

The X-ray generator 1312 receives a voltage and a current from a high voltage generator (HVG) to generate and emit X-rays.

The CT system 1300 may be implemented as a single-source CT system including one X-ray source 210 and one X-ray detector 220, a dual-source CT system including two X-ray sources, e.g., the first and second X-ray sources 711 and 712, and two X-ray detectors, e.g., the first and second X-ray detectors 721 and 722, or a dual-source CT system including two X-ray sources, e.g., the first and second X-ray sources 711 and 712, and a single X-ray detector 220.

The X-ray detector 220 detects radiation that has passed through the object. For example, the X-ray detector 220 may detect radiation by using a scintillator, a photon counting detector, etc.

Methods of driving the X-ray generator 1312 and the X-ray detector 220 may vary depending on scan modes used for scanning of the object. The scan modes are classified into an axial scan mode and a helical scan mode, according to a path along which the X-ray detector 220 moves. The scan modes are classified into a prospective mode and a retrospective mode, according to a time interval during which X-rays are emitted.

The controller 1330 may control an operation of each of the components of the CT system 1300. The controller 1330 may include a memory configured to store program codes for performing a function or data and a processor (e.g., a processor 240 or 740) configured to process the program codes or the data. The controller 1330 may be implemented in various combinations of at least one memory and at least one processor. The processor may generate or delete a program module according to an operating status of the CT system 1300 and process operations of the program module.

The readout device 1315 receives a detection signal generated by the X-ray detector 220 and outputs the detection signal to the image processor 1350. The readout device 1315 may include a DAS 1315-1 and a data transmitter 1315-2. The DAS 1315-1 uses at least one amplifying circuit to amplify a signal output from the X-ray detector 220, and outputs the amplified signal. The data transmitter 1315-2 uses a circuit such as a multiplexer (MUX) to output the signal amplified in the DAS 1315-1 to the image processor 1350. According to a slice thickness or a number of slices, only some of a plurality of pieces of data collected by the X-ray detector 220 may be provided to the image processor 1350, or the image processor 1350 may select only some of the plurality of pieces of data.

The image processor 1350 obtains tomography data from a signal obtained by the readout device 1315 (e.g., pure data that is data before being processed). The image processor 1350 may pre-process the obtained signal, convert the obtained signal into tomography data, and post-process the tomography data. The image processor 1350 may perform some or all of the processes described herein, and the type or order of processes performed by the image processor 1350 may vary according to embodiments.

The image processor 1350 may perform pre-processing, such as a process of correcting sensitivity irregularity between channels, a process of correcting a rapid decrease of signal strength, or a process of correcting signal loss due to an X-ray absorbing material, on the signal obtained by the readout device 1315.

According to embodiments, the image processor 1350 may perform some or all of the processes for reconstructing a tomography image, to generate the tomography data. According to an embodiment, the tomography data may be data that has undergone back-projection, or a tomography image. According to embodiments, additional processing may be performed on the tomography data by an external device such as a server, a medical apparatus, or a portable device.

Raw data is a set of data values corresponding to intensities of X-rays that have passed through the object, and may include projection data or a sinogram. The data that has undergone back-projection is obtained by performing back-projection on the raw data by using information about an angle at which X-rays are emitted. The tomography image is obtained by using image reconstruction techniques including back-projection of the raw data.

The storage 1340 is a storage medium for storing control-related data, image data, etc., and may include a volatile or non-volatile storage medium and/or a memory.

The input interface 1360 receives control signals, data, etc., from a user. The display 1370 may display information indicating an operational status of the CT system 1300, medical information, medical image data, etc., and may serve as a user input device if the display 1370 includes touchscreen.

The CT system 1300 includes the communication interface 1380 and may be connected to external devices, such as a server, a medical apparatus, and a portable device (smartphone, tablet personal computer (PC), wearable device, etc.), via the communication interface 1380.

The communication interface 1380 may include one or more components that enable communication with an external device. For example, the communication interface 1380 may include a short distance communication module, a wired communication module, and a wireless communication module.

The communication interface 1380 may receive control signals and data from an external device and transmit the received control signals to the controller 1330 so that the controller 1330 may control the CT system 1300 according to the received control signals.

Alternatively, by transmitting a control signal to an external device via the communication interface 1380, the controller 1330 may control the external device according to the control signal.

For example, the external device may process data according to a control signal received from the controller 1330 via the communication interface 1380.

A program for controlling the CT system 1300 may be installed on the external device and may include instructions for performing some or all of the operations of the controller 1330.

The program may be preinstalled on the external device, or a user of the external device may download the program from a server that provides an application for installation. The server that provides an application may include a recording medium having the program recorded thereon.

The above-described embodiments of the disclosure may be embodied as a computer-readable recording medium for storing computer executable command languages and data. The command languages may be stored as program codes and, when executed by a processor, may perform a certain operation by generating a certain program module. Also, when executed by a processor, the command languages may perform certain operations of embodiments.

While embodiments of the disclosure have been particularly shown and described with reference to the accompanying drawings, it will be understood by those of ordinary skill in the art that various changes in form and details may be made therein without departing from the spirit and scope of the disclosure as defined by the appended claims. The embodiments should be considered in descriptive sense only and not for purposes of limitation.

## Claims

1. A method of performing computed tomography (CT) imaging by injecting a contrast medium into an object, the method comprising:
setting a region of interest (ROI) in a scout image of the object;
acquiring X-ray projection images by detecting X-rays that have passed through the ROI;
determining a difference between pixel values of at least one first X-ray projection image acquired before an injection of the contrast medium into the ROI and pixel values of at least one second X-ray projection image acquired after the injection of the contrast medium into the ROI, among the X-ray projection images; and
determining whether to initiate a diagnostic CT scan by comparing the difference with a preset threshold.

2. The method of claim 1, wherein the determining the difference comprises:
setting, as a reference value, a sum of the pixel values of the at least one first X-ray projection image acquired before the injection of the contrast medium into the ROI;
calculating the difference between the reference value and a sum of the pixel values of the at least one second X-ray projection image acquired at each of a plurality of time points after the injection of the contrast medium into the ROI; and
determining, among the plurality of time points, a time point when the calculated difference exceeds the preset threshold,
wherein the determining whether to initiate the diagnostic CT scan comprises determining initiation of the diagnostic CT scan for examining the object at the determined time point.

3. The method of claim 2, wherein the acquiring the X-ray projection images comprises acquiring a plurality of first X-ray projection images at a plurality of angular positions arranged around the object, before the injection of the contrast medium into the ROI, the at least one first X-ray projection image being one of the plurality of first X-ray projection images,
the setting the reference value comprises setting, as the reference value, a sum of the pixel values of an X-ray projection image, among the plurality of first X-ray projection images, that is acquired at a first angular position among the plurality of angular positions, and
the calculating the difference comprises adding up, for each of the plurality of time points, the pixel values of the at least one second X-ray projection image that has been acquired at the first angular position at the plurality of time points after the injection of the contrast medium into the ROI, and calculating the difference between a result of the adding up and the reference value.

4. The method of claim 1, wherein the acquiring the X-ray projection images comprises:
detecting the X-rays that have passed through the ROI by a photon counting detector configured to detect the X-rays having a plurality of energy bands.

5. The method of claim 4, wherein the acquiring the X-ray projection images further comprises:
detecting, by the photon counting detector, the X-rays having an energy corresponding to a first energy band among the plurality of energy bands and acquiring the at least one second X-ray projection image based on the X-rays corresponding to the first energy band.

6. The method of claim 4, wherein the acquiring the X-ray projection images further comprises:
detecting the X-rays having an energy band corresponding to a k-edge that is in an energy band, in which an attenuation coefficient of the contrast medium increases rapidly, among the plurality of energy bands, and acquiring the at least one second X-ray projection image based on the X-rays corresponding to the k-edge.

7. The method of claim 4, wherein the acquiring the X-ray projection images further comprises:
emitting the X-rays from a plurality of X-ray sources having different tube voltages; and
acquiring a plurality of second X-ray projection images respectively corresponding to the plurality of energy bands by detecting, via a plurality of X-ray detectors, the X-rays that have passed through the ROI, the at least one second X-ray projection image being one of the plurality of second X-ray projection images,
wherein the determining the difference comprises:
calculating the difference between a reference value, which is a sum of the pixel values of the at least one first X-ray projection image acquired before the injection of the contrast medium into the ROI, and a sum of the pixel values of an X-ray projection image acquired, among the plurality of second X-ray projection images, by detecting the X-rays having an energy corresponding to an energy band in which an attenuation coefficient of the contrast medium undergoes a largest variation.

8. The method of claim 1, wherein the acquiring the X-ray projection images comprises:
separating, from the at least one second X-ray projection image, a contrast projection image generated by detecting the X-rays that have passed through the contrast medium,
wherein the determining the difference comprises:
setting, as a reference value, a sum of the pixel values of the at least one first X-ray projection image acquired before the injection of the contrast medium into the ROI; and
calculating the difference between a sum of the pixel values of the contrast projection image and the reference value.

9. A computed tomography (CT) apparatus comprising:
an X-ray source configured to emit X-rays toward a region of interest (ROI) of an object;
an X-ray detector configured to detect the X-rays that have passed through the ROI;
a data acquisition unit configured to acquire X-ray projection images based on the X-rays detected by the X-ray detector; and
a processor configured to set the ROI in a scout image of the object, determine a difference between pixel values of at least one first X-ray projection image acquired before an injection of a contrast medium into the ROI and pixel values of at least one second X-ray projection image acquired after the injection of the contrast medium into the ROI, among the X-ray projection images, and determine whether to initiate a diagnostic CT scan by comparing the difference with a preset threshold.

10. The CT apparatus of claim 9, wherein the processor is further configured to set, as a reference value, a sum of the pixel values of the at least one first X-ray projection image acquired before the injection of the contrast medium into the ROI, calculate the difference between the reference value and a sum of the pixel values of the at least one second X-ray projection image acquired at each of a plurality of time points after the injection of the contrast medium into the ROI, and determine initiation of the diagnostic CT scan for examining the object at a time point when the calculated difference exceeds the preset threshold among the plurality of time points.

11. The CT apparatus of claim 9, wherein the X-ray detector is a photon counting detector configured to detect the X-rays having a plurality of energy bands.

12. The CT apparatus of claim 11, wherein the X-ray detector is further configured to detect the X-rays having an energy corresponding to a first energy band among the plurality of energy bands, and
wherein the data acquisition unit is further configured to acquire the at least one second X-ray projection image based on the X-rays corresponding to the first energy band.

13. The CT apparatus of claim 11, wherein the X-ray detector is further configured to detect the X-rays having an energy band corresponding to a k-edge that is in an energy band, in which an attenuation coefficient of the contrast medium increases rapidly, among the plurality of energy bands, and
wherein the data acquisition unit is further configured to acquire the at least one second X-ray projection image based on the X-rays corresponding to the k-edge.

14. The CT apparatus of claim 11, wherein the X-ray source comprises a plurality of X-ray sources configured to emit the X-rays at different tube voltages,
wherein the X-ray detector comprises a plurality of X-ray detectors configured to detect the X-rays that are respectively emitted by the plurality of X-ray sources and pass through the ROI,
wherein the data acquisition unit is further configured to acquire a plurality of second X-ray projection images respectively corresponding to the plurality of energy bands based on the X-rays detected by the plurality of X-ray detectors, the at least one second X-ray projection image being one of the plurality of second X-ray projection images, and
wherein the processor is further configured to determine the difference between the pixel values for an X-ray projection image, which is acquired, among the plurality of second X-ray projection images, by detecting the X-rays having an energy corresponding to an energy band in which an attenuation coefficient of the contrast medium undergoes a largest variation, and the pixel values for the at least one first X-ray projection image acquired before the injection of the contrast medium into the ROI.

15. A computer program product comprising a computer-readable recording medium, wherein the computer-readable recording medium includes instructions for performing a method comprising:
setting a region of interest (ROI) in a scout image of an object;
acquiring X-ray projection images by detecting X-rays that have passed through the ROI;
determining a difference between pixel values of at least one X-ray projection image acquired before an injection of a contrast medium into the ROI and pixel values of at least one X-ray projection image acquired after the injection of the contrast medium into the ROI, among the X-ray projection images; and
determining whether to initiate a diagnostic CT scan by comparing the difference with a preset threshold.
